(19) Europäisches Patentamt
European Patent Office
Office européen des brevets

(11) EP 3 466 540 B1

(12) EUROPEAN PATENT SPECIFICATION

(45) Date of publication and mention of the grant of the patent:
**06.04.2022 Bulletin 2022/14**

(21) Application number: **18803852.5**

(22) Date of filing: **02.02.2018**

(51) International Patent Classification (IPC):
***B01J 31/18*** (2006.01) ***B01J 31/20*** (2006.01)
***B01J 31/22*** (2006.01) ***B01J 35/00*** (2006.01)
***B01J 23/40*** (2006.01) ***B01J 23/70*** (2006.01)
***B01J 31/12*** (2006.01) ***C07C 45/50*** (2006.01)
***C07C 47/02*** (2006.01)

(52) Cooperative Patent Classification (CPC):
(C-Sets available)
**B01J 31/185; B01J 31/20; B01J 31/2234; C07C 45/50;** B01J 31/121; B01J 2231/321; B01J 2531/822; B01J 2531/827; B01J 2531/845
(Cont.)

(86) International application number:
**PCT/KR2018/001429**

(87) International publication number:
**WO 2018/221830 (06.12.2018 Gazette 2018/49)**

(54) **CATALYST COMPOSITION FOR HYDROFORMYLATION REACTION AND METHOD FOR PREPARING ALDEHYDE USING SAME**

KATALYSATORZUSAMMENSETZUNG FÜR HYDROFORMULIERUNGSREAKTION UND VERFAHREN ZUR HERSTELLUNG VON ALDEHYD DAMIT

COMPOSITION DE CATALYSEUR POUR UNE RÉACTION D’HYDROFORMYLATION, ET PROCÉDÉ DE PRÉPARATION D’ALDÉHYDE L’UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2017 KR 20170066187**
**14.11.2017 KR 20170151202**

(43) Date of publication of application:
**10.04.2019 Bulletin 2019/15**

(73) Proprietor: **LG Chem, Ltd.**
**Seoul 150-721 (KR)**

(72) Inventors:
- **KIM, Tae Yun**
  **Daejeon 34122 (KR)**
- **CHOI, Min Ji**
  **Daejeon 34122 (KR)**
- **EOM, Sung Shik**
  **Daejeon 34122 (KR)**
- **KIM, Mi Young**
  **Daejeon 34122 (KR)**
- **KO, Dong Hyun**
  **Daejeon 34122 (KR)**
- **JUNG, Da Won**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**EP-A1- 3 156 127** **EP-A1- 3 482 827**
**WO-A1-2018/008928** **KR-A- 20180 006 299**
**KR-B1- 101 150 557** **US-A1- 2012 253 080**
**US-A1- 2012 253 080** **US-A1- 2012 259 142**
**US-B2- 9 221 850**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- **VAN ROOY, A. et al.: "Hydroformylation with a Rhodium/Bulky Phosphite Modified Catalyst. Catalyst Comparison for Oct-1-ene, Cyclohexene, and Styrene", Organometallics, vol. 14, no. 1, 1995, pages 34-43, XP055562602,**
- **KUBIS, C. et al.: "A Comparative In Situ HP-FTIR Spectroscopic Study of Bi-and Monodentate Phosphite-Modified Hydroformylation", ChemCatChem, vol. 2, no. 3, 8 March 2010 (2010-03-08), pages 287-295, XP055475830,**

(52) Cooperative Patent Classification (CPC): (Cont.)

C-Sets
**C07C 45/50**

## Description

[Technical Field]

The present invention relates to a catalyst composition for hydroformylation and a method of preparing an aldehyde using the same. More specifically, the present invention relates to a catalyst composition for hydroformylation including a specific phosphite-based compound and a transition metal catalyst and a method of preparing an aldehyde having excellent selectivity to iso-aldehyde along with excellent catalyst activity or stability using the catalyst composition.

[Background Art]

Hydroformylation, also known as oxo reaction, is a reaction of reacting various olefinic compounds with carbon monoxide (CO) and hydrogen ($H_2$), often referred to as synthesis gas, in the presence of a metal catalyst and a ligand to generate n-aldehyde and iso-aldehyde, the number of carbons of which is increased by one.

Aldehydes generated by oxo reaction may be oxidized or hydrogenated to be transformed into aldehyde derivatives, acids and alcohols, or may be condensation-reacted with aldol, etc. and then oxidized or hydrogenated to be transformed into various acids and alcohols containing long alkyl groups. Hydrogenated alcohols of aldehydes prepared by such oxo reaction are called oxo alcohols. These oxo alcohols are widely used for industrial purposes such as various solvents, additives, raw materials for plasticizers, and synthetic lubricants.

Conventionally, the value of n-aldehyde among products of hydroformylation was high, whereby most research on catalysts has been conducted to increase the proportion of n-aldehyde. However, recently, demand for iso-aldehyde is increasing due to development of, for example, isobutyric acid, neopentyl glycol (NPG), 2,2,4-trimethyl-1,3-pentanediol, and isovaleric acid prepared using iso-aldehyde derivatives as raw materials, whereby there is a need for development of a catalyst system having excellent reaction activity and stability while increasing the proportion of iso-aldehyde.

(Prior Art Documents)

(Patent Document) (Patent Document 1) JP 2013-515061 A
(Patent Document 2) KR 10- 1150557 B1
(Patent Document 3) EP-A-3156127 discloses catalyst compositions for hydroformylation comprising a mixture of a monodentate phosphite and a monodentate phosphine ligands, and a transition metal catalyst.

[Disclosure]

[Technical Problem]

Therefore, the present invention has been made in view of the above problems, and it is one object of the present invention to provide a catalyst composition for hydroformylation exhibiting excellent catalyst stability and activity despite use of a small amount of expensive transition metal catalyst compared to conventional cases.

It is another object of the present invention to provide a method of preparing aldehyde, the method being capable of providing iso-aldehyde in high yield due to a low N/I selectivity of 1.7 or less by reacting an olefinic compound with syngas ($CO/H_2$) in the presence of the catalyst composition.

The above and other objects can be accomplished by the present disclosure described below.

[Technical Solution]

In accordance with one aspect of the present invention, provided is a catalyst composition for hydroformylation as defined in and by the appended claims.

In accordance with another aspect of the present invention, provided is a method of preparing aldehyde, the method including a hydroformylation step of reacting an olefinic compound with syngas ($CO/H_2$) in the presence of the catalyst composition to prepare aldehyde.

[Advantageous effects]

As apparent from the foregoing, the present invention advantageously provides a catalyst composition for hydroformylation exhibiting high activity and maintaining high activity over a long period of time despite use of a small

amount of expressive transition metal compound at a level of 30 % as compared to conventional cases and, accordingly, having economic advantages. In addition, aldehyde obtained by applying the catalyst composition to a hydroformylation process of olefin exhibits an N/I selectivity of 1.7 or less, thereby generating iso-aldehyde in high yield.

[Description of Drawings]

FIG. 1 is graphs illustrating stability (productivity) test results of catalyst compositions according to examples and comparative examples.

[Best mode]

Hereinafter, a catalyst composition for hydroformylation of the present invention is described in detail.

The present inventors attempted to address the problem that stability is decreased and, accordingly, the lifespan of a catalyst is shortened although selectivity to iso-aldehyde is high and high activity is exhibited in an initial stage in the case of a conventional catalyst system including a rhodium catalyst and a phosphite-based ligand. As a result, the present inventors confirmed that, when a specific phosphite-based ligand and a rhodium catalyst are used in a specific amount range, the expensive rhodium catalyst may be used in a small content compared to conventional cases, the activity of the catalyst may be highly maintained, long-term stability may be improved, and, when the specific phosphite-based ligand and the rhodium catalyst, which are used in a specific amount range, are used in hydroformylation of propene, excellent iso-butyraldehyde selectivity is exhibited. Based on these findings, the present inventors continued further study and completed the present invention.

The catalyst composition for hydroformylation of the present invention is defined in the appended claims and includes a phosphite-based ligand represented by Formula 1 below; a transition metal compound; and a solvent, wherein the transition metal is included in a content of 50 to 90 ppm based on a total weight of the phosphite-based ligand, the transition metal compound, and the solvent.

[Formula 1]

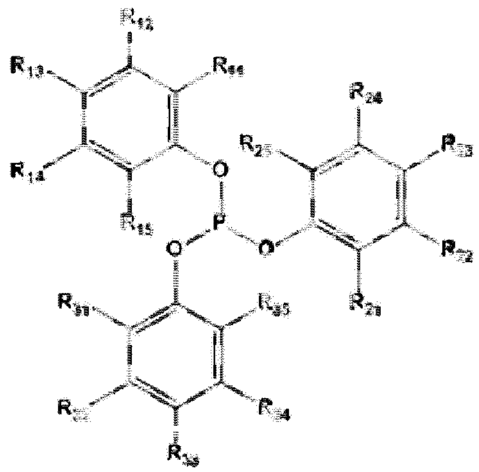

In Formula 1, $R_{12}$ , $R_{14}$ and $R_{15}$; $R_{22}$, $R_{24}$ and $R_{25}$; and $R_{32}$, $R_{34}$ and $R_{35}$ are the same or different and are each independently selected from among hydrogen, normal alkyl groups having 1 to 20 carbon atoms, and branched alkyl groups having 4 to 20 carbon atoms.

According to the invention in the phosphite-based ligand represented by Formula 1, each of $R_{11}$, $R_{13}$, $R_{21}$, $R_{23}$, $R_{31}$, and $R_{33}$ is a branched alkyl having 4 to 10 carbon atoms. More preferably, each of $R_{11}$, $R_{13}$, $R_{21}$, $R_{23}$, $R_{31}$, and $R_{33}$ is tert-butyl (t-Bu). In this case, when the phosphite-based ligand is applied to hydroformylation of an olefin compound, selectivity to iso-aldehyde may be excellent, and stability or activity of the catalyst may be excellent.

As a more specific example, the phosphite-based ligand represented by Formula 1 may be tris(2,4-di-tert-butylphenyl)phosphite represented by Formula 1a below . In this case even when the transition metal compound is used in a small amount, activity or stability of the catalyst may be highly maintained, and, when the phosphite-based ligand represented by Formula 1 is used in hydroformylation of olefin, excellent selectivity to iso-aldehyde may exhibited:

[Formula 1a]

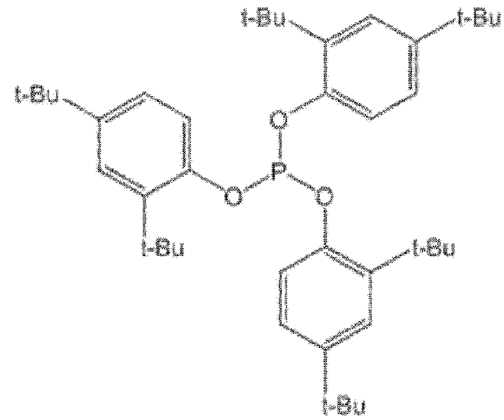

The phosphite-based ligand represented by Formula 1 is included in an amount of, 2 to 8 % by weight, 3 to 6 % by weight, 3 to 5 % by weight, 3 to 4.5 % by weight, 4 to 7 % by weight, or 4 to 6 % by weight, based on 100 % by weight of a catalyst composition including the phosphite-based ligand, a transition metal compound, and a solvent. Within these ranges, stability, activity, etc. of the catalyst may be highly maintained while lowering a use amount of the transition metal compound and, when the catalyst composition is used in hydroformylation, excellent selectivity to iso-aldehyde may be exhibited.

The transition metal compound is a compound represented by Formula 2 below:

[Formula 2] $M(L^1)_x(L^2)_y(L^3)_z$

wherein M is one selected from the group consisting of rhodium (Rh), cobalt (Co), iridium (Ir), ruthenium (Ru), iron (Fe), nickel (Ni), palladium (Pd), platinum (Pt), and osmium (Os), $L^1$, $L^2$, and $L^3$ are each independently one selected from the group consisting of hydrogen, carbonyl (CO), cyclooctadiene, norbornene, chlorine, triphenylphosphine (TPP), and acetylacetonate (AcAc), x, y, and z are each independently an integer of 0 to 5, and x, y and z are not 0 at the same time.

As a more specific example, the transition metal compound may be one or more selected from the group consisting of (acetylacetonato)dicarbonylrhodium [Rh (AcAc) $(CO)_2$], (acetylacetonato)carbonyl(triphenylphosphine)rhodium [Rh(AcAc)(CO) (TPP)], hydridocarbonyltris(triphenylphosphine)rhodium [HRh(CO) $(TPP)_3$], (acetylacetonato)carbonyliridium [Ir(AcAc)$(CO)_2$], and hydridocarbonyltris(triphenylphosphine)iridium [HIr(CO) $(TPP)_3$] , but the present invention is not limited thereto.

According to the invention the transition metal is included in an amount of 50 to 90 ppm, 50 to 80 ppm, 60 to 80 ppm, or 70 to 80 ppm based on a total weight of a catalyst composition including the phosphite-based ligand represented by Formula 1, a transition metal compound, and a solvent. Within these ranges, activity or stability of the catalyst may be high while using a small amount of expensive transition metal compound.

A molar ratio (L/M) of the phosphite-based ligand to the transition metal compound included in the catalyst composition of the present invention may be, for example, 32 to 320, 35 to 320, 40 to 238, 60 to 238, 60 to 230, 70 to 230, 100 to 230, 120 to 230, or 150 to 230. In this case, stability or activity of the catalyst may be highly maintained while lowering a use amount of the transition metal compound and, when the catalyst composition is used in hydroformylation, excellent selectivity to iso-aldehyde may be exhibited.

The catalyst composition of the present invention is a composition free from one coordinated phosphine ligand. In this case, stability, activity, etc. of the catalyst may be excellent and excellent selectivity to iso-aldehyde may be provided while greatly lowering a use amount of the transition metal compound compared to conventional cases.

In the present invention, the expression "free from one coordinated phosphine ligand" refers to the case wherein one coordinated phosphine ligand is intentionally omitted.

The catalyst composition of the present invention is a single ligand system only including the phosphite-based ligand represented by Formula 1 as a ligand. In this case, costs may be considerably reduced by lowering the content of the transition metal compound, activity, stability, etc. of the catalyst may be excellent, and excellent selectivity to iso-aldehyde may be exhibited.

As a most preferable example, the catalyst composition of the present invention includes tris(2,4-di-tert-butylphenyl)phosphite as the phosphite-based ligand represented by Formula 1 and is a composition free from one coordinated phosphine ligand. In this case, despite application of a small amount of transition metal compound, activity or stability of the catalyst is considerably excellent and excellent selectivity to iso-aldehyde is exhibited.

The catalyst composition of the present invention includes the phosphite-based ligand represented by Formula

1; the transition metal compound represented by Formula 2; and a solvent. Here, the content of the transition metal is from 50 to 90 ppm based on a total weight of the phosphite-based ligand, the transition metal compound, and the solvent. In this case, a use amount of the expensive transition metal compound is greatly lowered, and stability and activity of the catalyst and selectivity to iso-aldehyde are excellent.

Here, the phosphite-based ligand represented by Formula 1 is preferably tris(2,4-di-tert-butylphenyl)phosphite in terms of activity or stability of a catalyst or selectivity to iso-aldehyde.

The solvent may be, for example, one or more selected from the group consisting of propionaldehyde, butyraldehyde, pentyl aldehyde, acetone, methyl ethyl ketone, methyl isobutyl ketone, acetophenone, cyclohexanone, ethanol, pentanol, octanol, texanol, benzene, toluene, xylene, orthodichlorobenzene, tetrahydrofuran, dimethoxyethane, dioxane, methylene chloride, and heptane.

As a preferred example, the solvent is an aldehyde such as propionaldehyde, butyraldehyde, or pentyl aldehyde. More preferably, a compound the same as the aldehyde generated by hydroformylation is used. In this case, a product may be easily isolated and improved purity is provided.

The catalyst composition for hydroformylation may be, for example, a catalyst composition used to produce butyraldehyde from propene.

As a specific example, the catalyst composition may generate n-butyraldehyde and iso-butyraldehyde in a molar ratio of 1.7 or less, 1.0 to 1.7, 1.5 or less, 1.2 to 1.5, or 1.2 to 1.3. That is, the catalyst composition may generate iso-butyraldehyde in high yield.

The catalyst composition may be used in hydroformylation. Hereinafter, a method of preparing aldehyde by reacting an olefinic compound with syngas in the presence of the catalyst composition for hydroformylation is described.

The method of preparing aldehyde of the present invention may include a hydroformylation step of reacting an olefinic compound with syngas ($CO/H_2$) in the presence of the catalyst composition for hydroformylation to prepare aldehyde.

In the present invention, the olefinic compound may be, for example, a compound represented by Formula 3 below:

[Formula 3]

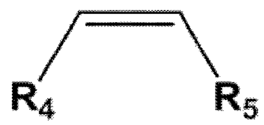

wherein $R_4$ and $R_5$ are each independently hydrogen, an alkyl group having 1 to 20 carbon atoms, fluorine (-F), chlorine (-Cl), bromine (-Br), a trifluoromethyl group ($-CF_3$), an unsubstituted C6 to C20 aryl group, or a C6 to C20 aryl group substituted with 1 to 5 substituents. Here, the substituents of the aryl group are selected from among a nitro group ($-NO_2$), fluorine (-F), chlorine (-Cl), bromine (-Br), and a C1 to C5 alkyl group.

As a specific example, the olefinic compound may be one or more selected from the group consisting of ethene, propene, 1-butene, 1-pentene, 1-hexene, 1-octene, and styrene, but the present invention is not limited thereto.

As a more preferable example, the olefinic compound may be propene, and the aldehyde may be butyraldehyde. When the catalyst composition according to the present invention is used in hydroformylation of propene, a molar ratio of n-butyraldehyde to iso-butyraldehyde is low, i.e., the molar ratio is 1.7 or less, or 1.5 or less. Accordingly, iso-butyraldehyde may be generated in high yield.

A molar ratio of carbon monoxide (CO) to hydrogen ($H_2$) in the syngas may be, for example, 5 : 95 to 70 : 30, 40 : 60 to 60 : 40, or 45 : 55 to 55 : 45. Within these ranges, the gas used for the reaction is not accumulated in a reactor and excellent reaction balance is exhibited.

In the present invention, the hydroformylation is preferably carried out, for example, at 20 to 180 °C, 50 to 150 °C, 60 to 125 °C, 70 to 100 °C, 70 to 90 °C, or 80 to 90 °C. Within these ranges, stability and activity of the catalyst are excellent, the reaction may rapidly proceed, and side reactions, such as decomposition of a ligand, may be minimized.

In addition, the hydroformylation of the present invention is preferably carried out, for example, under a pressure condition of 1 to 100 bar, 1 to 500 bar, 5 to 30 bar, 5 to 13 bar, 5 to 9 bar, or 14 to 20 bar. Within these ranges, the risk of explosion is low, and aldehyde may be obtained in high yield due to rapid hydroformylation.

Hereinafter, the present invention will be described in more detail with reference to the following preferred examples. However, these examples are provided for illustrative purposes only and should not be construed as limiting the scope of the present invention. In addition, it will be apparent to those skilled in the art that various changes and modifications may be made without departing from the scope of the present invention, and such changes and modifications

are also within the scope of the appended claims.

[Example]

Example 1

1. Preparation of catalyst composition

0.024 g (0.049 mmol) of (acetylacetonato)dicarbonyl triphenyl rhodium [Rh(AcAc)(CO)(TPP), ROPAC], as a catalyst, and tris(2,4-di-tert-butylphenyl)phosphite (TDTBPP), as a ligand, were dissolved in a butyraldehyde solvent such that a total weight of resultant solution reached 100.5 g (50 ppm of Rh, 6 % by weight of TDTBPP), followed by feeding the same into an autoclave. The air was replaced with nitrogen while stirring the reaction solution. Subsequently, a gas mixture prepared by mixing propene and syngas ($CO/H_2$) in a molar ratio of 1 : 1 : 1 ($C_3H_6$ : CO : $H_2$) was injected into the reaction solution, the pressure in the autoclave was maintained at 8 bar, and reaction was carried out while stirring at 90 °C for 1 hour.

Examples 2 to 4, Examples not according to the invention 5-8, Comparative Examples 1 to 7, and Reference Examples 1 to 4

Experiments were carried out in the same manner as in Example 1, except that the content of ROPAC and the content and type of ligand were changed as summarized in Table 1 below.

[Test Example]

N/I selectivity, catalyst activity, and stability of aldehyde prepared according to each of Examples 1 to 8, Comparative Examples 1 to 7, and Reference Examples 1 to 4 were measured according to the following methods. Results are summarized in Tables 1 and 2 below and illustrated in the accompanying FIG. 1.

Test Example 1: N/I selectivity

The amount of n-butyraldehyde produced by hydroformylation was divided by the amount of iso-butyraldehyde. A generation amount of each aldehyde was determined by gas chromatography (GC).

Test Example 2: Activity of catalyst

A total weight of n-butyraldehyde and iso-butyraldehyde produced through reaction using the catalyst according to each of the examples, the comparative examples, and the reference examples was compared to 100 % of a total weight of n-butyraldehyde and iso-butyraldehyde produced through reaction (reaction pressure: 8 bar, reaction temperature: 90 °C) using the catalyst according to Comparative Example 1. Catalyst activity was calculated according to Equation 1 below, and represented as a percentage:

[Equation 1]

Catalyst activity (%) = (Total weight of n-butyraldehyde and iso-butyraldehyde according to example, comparative example, or reference example / total weight of n-butyraldehyde and iso-butyraldehyde according to Comparative Example 1) X 100

Examples 5-8 are not according to the invention

[Table 1]

| Classification | Ligand type | Ligand content (% by weight) | Rhodium use amount (ppm) | N/I selectivity | Catalyst activity (%) |
|---|---|---|---|---|---|
| Example 1 | TDTBPP | 3 | 50 | 1.2 ~1.6 | 788 |
| Example 2 | TDTBPP | 6 | 50 | 1.2 ~ 1.6 | 788 |
| Example 3 | TDTBPP | 4.5 | 75 | 1.2 ~ 1.6 | 863 |
| Example 4 | TDTBPP | 6 | 75 | 1.2 ~ 1.6 | 846 |
| Example 5 | TDTBPP | 3 | 100 | 1.2 ~ 1.6 | 954 |
| Example 6 | TDTBPP | 6 | 100 | 1.2 ~ 1.6 | 1084 |
| Example 7 | TTBMPP | 3 | 50 | 1.3 ~ 1.7 | 680 |
| Example 8 | TTBMPP | 6 | 75 | 1.3 ~ 1.7 | 650 |
| Comparative Example 1 | CHDP + TPTP | 3[a)] | 250 | 3.5 | 100 |
| Comparative Example 2 | CHDP | 2 | 250 | 2.5 | 96 |
| Comparative Example 3 | TPTP | 3 | 250 | 6 | 130 |
| Comparative Example 4 | TDTBPP | 3 | 250 | 1.8 | 1200 |
| Comparative Example 5 | TDTBPP | 10 | 250 | 2 | 1178 |
| Comparative Example 6 | TTBMPP | 6 | 250 | 1.9 | 604 |
| Comparative Example 7 | TDTBPP | 6 | 30 | 1.3 | 270 |
| Reference Example 1 | TDTBPP + CHDP | 3[b)] | 250 | 2 | 105 |
| Reference Example 2 | TDTBPP+CHDP | 3[c)] | 100 | 2.2 | 60 |
| Reference Example 3 | TDTBPP+TPTP | 3[d)] | 250 | 2.4 | 225 |
| Reference Example 4 | TDTBPP+TPTP | 3[e)] | 100 | 2.6 | 76 |

a) : Mixture of 2 % by weight of CHDP and 1 % by weight of TPTP
b) : Mixture of 2 % by weight of TDTBPP and 1 % by weight of CHDP
c) : Mixture of 2 % by weight of TDTBPP and 1 % by weight of CHDP
d) : Mixture of 2 % by weight of TDTBPP and 1 % by weight of TPTP
e) : Mixture of 2 % by weight of TDTBPP and 1 % by weight of TPTP
** TTBMPP: Tris(2-tert-butyl-4-methylphenyl)phosphite
* CHDP: Cyclohexyldiphenylphosphine
* TPTP: Tri-p-tolylphosphine

As shown in Table 1, it can be confirmed that, when hydroformylation is carried out using the catalyst composition of the present invention, N/I selectivity is 1.7 or less, i.e., selectivity to iso butyraldehyde is high, and catalyst activity is excellent although the amount of rhodium is about 20 to 40 % of those of Comparative Examples 1 to 6.

In addition, it can be confirmed that, when the catalyst composition of each of Comparative Examples 4 to 6, in which TDTBPP or TTBMPP, as a ligand, is used in the same content as in the examples, but the content of rhodium

is 250 ppm, i.e., the rhodium content is excessively high, is used in hydroformylation, N/I selectivity exceeds a desired value. On the other hand, it can be confirmed that, when the catalyst composition of Comparative Example 7, in which the content of rhodium is 30 ppm, i.e., the rhodium content is low, is used in hydroformylation, selectivity to iso butyraldehyde is excellent, but catalyst activity is considerably decreased.

Meanwhile, it can be confirmed that, when TDTBPP or TTBMPP is included as a ligand, but one coordinated phosphine ligand, i.e., TPTP or CHDP, is mixed, N/I selectivity greatly exceeds a desired value, and catalyst activity is also considerably decreased. In addition, it can be confirmed that, when the content of rhodium is controlled to 100 ppm, (Reference Examples 2 and 4), stability is further decreased and thus very poor.

Test Example 3: catalyst stability (productivity)

Syngas ($CO/H_2$) mixed in a molar ratio of 1 : 1 was injected into the reaction solution until the pressure of a reactor reached 6.05 bar, followed by aging while stirring at 118.9 °C for 0 hours, 2.5 hours, 5 hours, 15 hours, and 24 hours, and then cooling to 20 °C. Subsequently, the syngas was substituted with a gas mixture of propene ($C_3H_6$) and syngas ($CO/H_2$) mixed in a molar ratio of 1 : 1 : 1 ($C_3H_6$ : CO : $H_2$) . Subsequently, reaction was allowed for one hour while maintaining the pressure inside the reactor at 8 bar and stirring at 90 °C, followed by calculating catalyst activity. Results are summarized in Table 2 below and the accompanying FIG. 1.

Examples 5-8 are not according to the invention

[Table 2]

| Classification | Catalyst stability (productivity) test | | | | |
|---|---|---|---|---|---|
| | 0 hr | 2.5 hr | 5 hr | 15 hr | 24 hr |
| Example 1 | 788 | 521 | 475 | 444 | 398 |
| Example 2 | 788 | 626 | 594 | 579 | 550 |
| Example 3 | 863 | 580 | 510 | 398 | 396 |
| Example 4 | 846 | 800 | 731 | 686 | 684 |
| Example 5 | 954 | 611 | 506 | 348 | 336 |
| Example 6 | 1084 | 914 | 888 | 808 | 492 |
| Example 7 | 680 | 518 | 457 | 434 | 387 |
| Example 8 | 650 | 602 | 592 | 586 | 580 |
| Comparative Example 1 | 100 | 70 | 58 | 49 | 43 |
| Comparative Example 2 | 96 | 87 | 59 | 52 | 52 |
| Comparative Example 3 | 130 | 66 | 5 | 40 | 28 |
| Comparative Example 4 | 1200 | 754 | 224 | 177 | 6 |
| Comparative Example 5 | 1178 | 771 | 392 | 277 | 167 |
| Comparative Example 6 | 604 | 363 | 195 | 161 | 85 |
| Comparative Example 7 | 270 | 150 | 120 | 115 | 94 |
| Reference Example 1 | 105 | 87 | 84 | 80 | 78 |
| Reference Example 2 | 60 | 54 | 53 | 51 | 50 |
| Reference Example 3 | 225 | 187 | 160 | 138 | 126 |
| Reference Example 4 | 76 | 60 | 58 | 50 | 46 |

As shown in Table 2 and FIG. 1, it can be confirmed that, although the content of rhodium in the catalyst compositions according to Examples 1 to 4 is about 20 to 40 % of those of Comparative Examples 1 to 6, initial catalyst activity is excellent, long-term stability of the catalyst is excellent, and, particularly, catalyst activity is 300 % or more,

i.e., catalyst activity is highly maintained, also after aging over a period of 24 hours.

In addition, it can be confirmed that, in the case of the catalyst composition of Comparative Example 7 in which rhodium is included in a smaller amount compared to the catalyst composition according to the present invention, catalyst activity is considerably decreased after aging, i.e., catalyst stability is poor.

On the other hand, it can be confirmed that, in the case of Comparative Examples 1 to 3 in which ligands not according to the present invention are used, initial catalyst activity and stability are poor although the content of rhodium is considerably excessive compared to the examples. In addition, it can be confirmed that, in the case of Comparative Example 4 to 6 in which a ligand not according to the present invention is used, the catalyst compositions exhibit excellent initial catalyst activity, but catalyst stability is rapidly decreased.

Further, it can be confirmed that, in the case of Reference Examples 1 to 4 in which a mixture of a phosphite-based ligand and one coordinated phosphine ligand is used, initial catalyst activity is very poor as described above, and catalyst activity is further decreased after aging over a period of 24 hours.

## Claims

1. A catalyst composition for hydroformylation, comprising:

   a phosphite-based ligand represented by Formula 1 below;
   a transition metal compound represented by Formula 2 below; and
   a solvent,
   wherein the transition metal is comprised in a content of 50 to 90 ppm based on a total weight of the phosphite-based ligand, the transition metal compound, and the solvent:

   [Formula 1]

   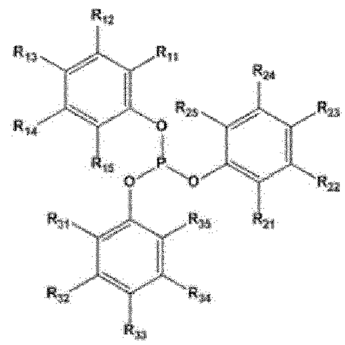

   wherein $R_{12}$, $R_{14}$ and $R_{15}$; $R_{22}$ $R_{24}$, and $R_{25}$; and $R_{32}$, $R_{34}$ and $R_{35}$ are the same or different and are each independently selected from among hydrogen, normal alkyl groups having 1 to 20 carbon atoms, and branched alkyl groups having 4 to 20 carbon atoms; and each of $R_{11}$, $R_{13}$, $R_{21}$, $R_{23}$, $R_{31}$, and $R_{33}$ is a branched alkyl group having 4 to 10 carbon atoms, and

   [Formula 2] $M (L^1)_x(L^2)_y(L^3)_z$

   wherein M is one selected from the group consisting of rhodium (Rh), cobalt (Co), iridium (Ir), ruthenium (Ru), iron (Fe), nickel (Ni), palladium (Pd), platinum (Pt), and osmium (Os), $L^1$, $L^2$, and $L^3$ are each independently one selected from the group consisting of hydrogen, carbonyl (CO), cyclooctadiene, norbornene, chlorine, triphenylphosphine (TPP), and acetylacetonate (AcAc), x, y, and z are each independently an integer of 0 to 5, and x, y and z are not 0 at the same time,

   wherein the catalyst composition is a single ligand system only including the phosphite-based ligand represented by formula 1, and
   wherein the phosphite-based ligand is comprised in an amount of 2 to 8 % by weight based on 100 % by weight of the sum of the phosphite-based ligand, the transition metal compound, and the solvent.

2. The catalyst composition according to claim 1, wherein a molar ratio (L/M) of the phosphite-based ligand to the transition metal compound is 32 to 320.

3. The catalyst composition according to claim 1, wherein the transition metal compound is one or more selected from the group consisting of (acetylacetonato)dicarbonylrhodium [Rh (AcAc) $(CO)_2$],

(acetylacetonato)carbonyl(triphenylphosphine)rhodium [Rh(AcAc)(CO)(TPP)], hydridocarbonyltris(triphenylphosphine)rhodium [HRh(CO) $(TPP)_3$], (acetylacetonato)carbonyliridium [Ir(AcAc)$(CO)_2$], and hydridocarbonyltris(triphenylphosphine)iridium [HIr(CO)(TPP) $_3$].

4. Use of the catalyst composition according to claim 1 to produce butyraldehyde from propene.

5. The use according to claim 4, wherein butyraldehyde comprises n-butyraldehyde and iso-butyraldehyde in a molar ratio of 1.7 or less.

6. The catalyst composition according to claim 1, wherein the solvent is one or more selected from the group consisting of propionaldehyde, butyraldehyde, pentyl aldehyde, acetone, methyl ethyl ketone, methyl isobutyl ketone, acetophenone, cyclohexanone, ethanol, pentanol, octanol, texanol, benzene, toluene, xylene, orthodichlorobenzene, tetrahydrofuran, dimethoxyethane, dioxane, methylene chloride, and heptane.

7. A method of preparing aldehyde, the method comprising a hydroformylation step of reacting an olefinic compound with syngas ($CO/H_2$) in the presence of the catalyst composition according to claim 1 to prepare aldehyde.

8. The method according to claim 7, wherein, in the syngas, carbon monoxide (CO) and hydrogen ($H_2$) are mixed in a molar ratio of 5 : 95 to 70 : 30.

9. The method according to claim 7, wherein the hydroformylation step is carried out under conditions of a reaction temperature of 20 to 180 °C and a pressure of 1 to 100 bar.

10. The method according to claim 7, wherein the olefinic compound is propene, and the aldehyde is butyraldehyde.

**Patentansprüche**

1. Katalysatorzusammensetzung zur Hydroformylierung, umfassend:

einen Liganden auf Basis von Phosphit, der durch die nachstehende Formel 1 dargestellt ist;
eine Übergangsmetallverbindung, die durch die nachstehende Formel 2 dargestellt ist; und
ein Lösungsmittel,
wobei das Übergangsmetall mit einem Gehalt von 50 bis 90 ppm, bezogen auf das Gesamtgewicht des Liganden auf Basis von Phosphit, der Übergangsmetallverbindung und des Lösungsmittels, enthalten ist:

[Formel 1]

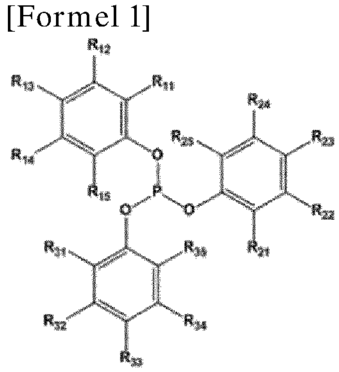

wobei $R_{12}$, R14 und R15; R22, R24 und R25; und R32, R34 und R35 gleich oder verschieden sind und jeweils unabhängig voneinander ausgewählt sind aus Wasserstoff, normalen Alkylgruppen mit 1 bis 20 Kohlenstoffatomen und verzweigten Alkylgruppen mit 4 bis 20 Kohlenstoffatomen; und R11, $R_{13}$, $R_{21}$, R23, R31 und R33 jeweils eine verzweigte Alkylgruppe mit 4 bis 10 Kohlenstoffatomen sind, und

[Formel 2] $M(L^1)_x(L^2)_y(L^3)_z$

wobei M eines ist, ausgewählt aus der Gruppe bestehend aus Rhodium (Rh), Cobalt (Co), Iridium (Ir), Ruthenium (Ru), Eisen (Fe), Nickel (Ni), Palladium (Pd), Platin (Pt) und Osmium (Os), $L^1$, $L^2$ und $L^3$ jeweils unabhängig voneinander ausgewählt sind aus der Gruppe bestehend aus Wasserstoff, Carbonyl (CO), Cyclooctadien, Norbornen, Chlor, Triphenylphosphin (TPP) und Acetylacetonat (AcAc), x, y und z jeweils unabhängig voneinander eine ganze Zahl von 0 bis 5 sind und x, y und z nicht gleichzeitig 0 sind,
wobei die Katalysatorzusammensetzung ein Einzelligandensystem ist, das nur den Liganden auf Basis von Phosphit, der durch die Formel 1 dargestellt ist, einschließt, und
wobei der Ligand auf Basis von Phosphit in einer Menge von 2 bis 8 Gew.-%, bezogen auf 100 Gew.-% der Summe des Liganden auf Basis von Phosphit, der Übergangsmetallverbindung und des Lösungsmittels, enthalten ist.

**2.** Katalysatorzusammensetzung nach Anspruch 1, bei der das Molverhältnis (L/M) des Liganden auf Basis von Phosphit zu der Übergangsmetallverbindung 32 bis 320 beträgt.

**3.** Katalysatorzusammensetzung nach Anspruch 1, bei der die Übergangsmetallverbindung eine oder mehrere ist, ausgewählt aus der Gruppe bestehend aus (Acetylacetonato)dicarbonylrhodium [$Rh(AcAc)(CO)_2$], (Acetylacetonato)carbonyl(triphenylphosphin)rhodium [Rh(AcAc)(CO)(TPP)], Hydridocarbonyltris(triphenylphosphin)rhodium [$HRh(CO)(TPP)_3$], (Acetylacetonato)carbonyliridium [$Ir(AcAc)(CO)_2$] und Hydridocarbonyl*tris*(triphenylphosphin)iridium [$HIr(CO)(TPP)_3$].

**4.** Verwendung der Katalysatorzusammensetzung nach Anspruch 1 zur Herstellung von Butyraldehyd aus Propen.

**5.** Verwendung nach Anspruch 4, bei der Butyraldehyd n-Butyraldehyd und iso-Butyraldehyd in einem Molverhältnis von 1,7 oder weniger umfasst.

**6.** Katalysatorzusammensetzung nach Anspruch 1, bei der das Lösungsmittel eines oder mehrere ist, ausgewählt aus der Gruppe bestehend aus Propionaldehyd, Butyraldehyd, Pentylaldehyd, Aceton, Methylethylketon, Methylisobutylketon, Acetophenon, Cyclohexanon, Ethanol, Pentanol, Octanol, Texanol, Benzol, Toluol, Xylol, Orthodichlorbenzol, Tetrahydrofuran, Dimethoxyethan, Dioxan, Methylenchlorid und Heptan.

**7.** Verfahren zur Herstellung von Aldehyd, wobei das Verfahren einen Hydroformylierungsschritt des Umsetzens einer olefinischen Verbindung mit Syngas ($CO/H_2$) in Gegenwart der Katalysatorzusammensetzung nach Anspruch 1 zur Herstellung von Aldehyd umfasst.

**8.** Verfahren nach Anspruch 7, bei dem im Syngas Kohlenmonoxid (CO) und Wasserstoff ($H_2$) in einem Molverhältnis von 5:95 bis 70:30 gemischt sind.

**9.** Verfahren nach Anspruch 7, bei dem der Hydroformylierungsschritt unter Bedingungen einer Reaktionstemperatur von 20 bis 180 °C und einem Druck von 1 bis 100 bar durchgeführt wird.

**10.** Verfahren nach Anspruch 7, bei dem die olefinische Verbindung Propen ist und der Aldehyd Butyraldehyd ist.

## Revendications

**1.** Composition de catalyseur pour hydroformylation, comprenant :

un ligand à base de phosphite représenté par la Formule 1 ci-dessous ;
un composé de métal de transition représenté par la Formule 2 ci-dessous ; et
un solvant,
dans laquelle le métal de transition est composé dans une teneur comprise entre 50 et 90 ppm sur la base du poids total du ligand à base de phosphite, du composé de métal de transition et du solvant :

[Formule 1]

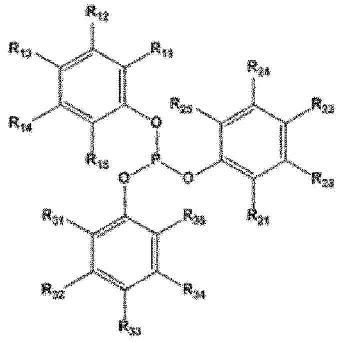

dans laquelle $R_{12}$, $R_{14}$ et $R_{15}$ ; $R_{22}$, $R_{24}$ et $R_{25}$ ; et $R_{32}$, $R_{34}$ et $R_{35}$ sont identiques ou différents et sont chacun indépendamment sélectionnés parmi un hydrogène, des groupes alkyle normaux ayant 1 à 20 atomes de carbone et des groupes alkyle ramifiés ayant 4 à 20 atomes de carbone ; et chacun de $R_{11}$, R13, $R_{21}$, $R_{23}$, $R_{31}$ et $R_{33}$ est un groupe alkyle ramifié ayant 4 à 10 atomes de carbone, et

[Formule 2] $M(L^1)_x(L^2)_y(L^3)_z$

dans laquelle M est sélectionné dans le groupe constitué de rhodium (Rh), cobalt (Co), iridium (Ir), ruthénium (Ru), fer (Fe), nickel (Ni), palladium (Pd), platine (Pt) et osmium (Os), $L^1$, $L^2$ et $L^3$ sont chacun indépendamment sélectionnés dans le groupe constitué d'un hydrogène, carbonyle (CO), cyclooctadiène, norbornène, chlore, triphénylphosphine (TPP) et acétylacétonate (AcAc), x, y, et z sont chacun indépendamment un nombre entier de 0 à 5, et x, y et z ne sont pas 0 en même temps,
dans laquelle la composition de catalyseur est un système à ligand unique seulement incluant le ligand à base de phosphite représenté par la formule 1, et
dans laquelle le ligand à base de phosphite est composé dans une quantité comprise entre 2 et 8 % en poids sur la base de 100 % en poids de la somme du ligand à base de phosphite, du composé de métal de transition et du solvant.

2. Composition de catalyseur selon la revendication 1, dans laquelle un rapport molaire (L/M) du ligand à base de phosphite au composé de métal de transition est compris entre 32 et 320.

3. Composition de catalyseur selon la revendication 1, dans laquelle le composé de métal de transition est un ou plusieurs éléments sélectionnés dans le groupe constitué d'acétylacétonate de rhodium dicarbonyle $[Rh(AcAc)(CO)_2]$, acétylacétonate de rhodium carbonyle (triphénylphosphine) [Rh(AcAc)(CO)(TPP)], hydridocarbonyle tris(triphénylphosphine) de rhodium $[HRh(CO)(TPP)_3]$, acétylacétonate d'iridium carbonyle $[Ir(AcAc)(CO)_2]$ et hydridocarbonyle tris(triphénylphosphine) d'iridium $[HIr(CO)(TPP)_3]$.

4. Utilisation de la composition de catalyseur selon la revendication 1 pour produire un butyraldéhyde à partir de propène.

5. Utilisation selon la revendication 4, dans laquelle le butyraldéhyde comprend un n-butyraldéhyde et un iso-butyraldéhyde dans un rapport molaire égal ou inférieur à 1,7.

6. Composition de catalyseur selon la revendication 1, dans laquelle le solvant est un ou plusieurs éléments sélectionnés dans le groupe constitué d'aldéhyde propionique, aldéhyde butyrique, aldéhyde pentylique, acétone, méthyléthylcétone, méthylisobutylcétone, acétophénone, cyclohexanone, éthanol, pentanol, octanol, texanol, benzène, toluène, xylène, orthodichlorobenzène, tétrahydrofurane, diméthoxyéthane, dioxane, chlorure de méthylène et heptane.

7. Procédé de préparation d'un aldéhyde, le procédé comprenant une étape d'hydroformylation pour faire réagir un composé oléfinique avec un gaz de synthèse $(CO/H_2)$ en présence de la composition de catalyseur selon la revendication 1 pour préparer un aldéhyde.

8. Procédé selon la revendication 7, dans laquelle, dans le gaz de synthèse, un monoxyde de carbone (CO) et un hydrogène $(H_2)$ sont mélangés dans un rapport molaire compris entre 5:95 et 70:30.

**9.** Procédé selon la revendication 7, dans laquelle l'étape d'hydroformylation est effectuée dans des conditions de température de réaction comprise entre 20 et 180°C et de pressure comprise entre 1 et 100 bar.

**10.** Procédé selon la revendication 7, dans laquelle le composé oléfinique est un propène, et l'aldéhyde est un butyraldéhyde.

【FIG. 1】

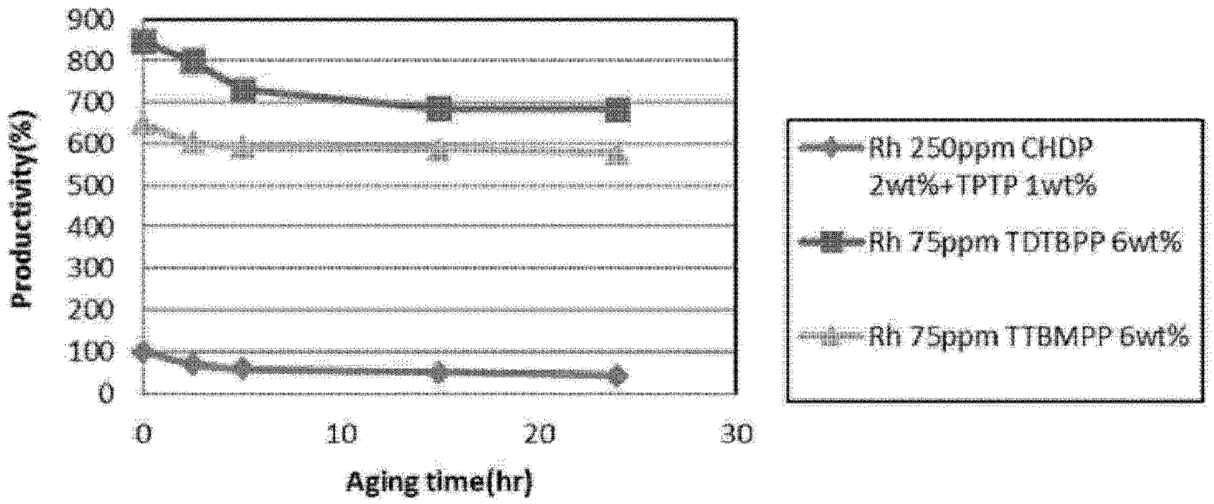
900
800
700
600
500
400
300
200
100
0
Productivity(%)
0
10
20
30
Aging time(hr)
Rh 250ppm CHDP 2wt%+TPTP 1wt%
Rh 75ppm TDTBPP 6wt%
Rh 75ppm TTBMPP 6wt%

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013515061 A
- KR 101150557 B1
- EP 3156127 A